Europäisches Patentamt

European Patent Office

Office européen des brevets

(19)

(11) Publication number: **0 035 780**
**B1**

(12) **EUROPEAN PATENT SPECIFICATION**

(45) Date of publication of patent specification: **25.01.84**

Int. Cl.³: **A 61 K 9/36**

(21) Application number: **81101663.3**

(22) Date of filing: **06.03.81**

(54) **Coating compositions for enterosoluble solid dosage forms.**

(30) Priority: **10.03.80 JP 30121/80**

(43) Date of publication of application:
**16.09.81 Bulletin 81/37**

(45) Publication of the grant of the patent:
**25.01.84 Bulletin 84/4**

(84) Designated Contracting States:
**CH DE FR GB LI**

(56) References cited:
**EP - A - 0 008 780**
**EP - A - 0 013 566**
**FR - A - 2 308 355**
**FR - A - 2 404 029**
**GB - A - 1 444 890**
**GB - A - 2 006 217**

(73) Proprietor: **Shin-Etsu Chemical Co., Ltd.**
**6-1, Ohtemachi 2-chome**
**Chiyoda-ku, Tokyo (JP)**

(72) Inventor: **Sekigawa, Fujio**
**1200-77, Horisaki-cho**
**Omiya-shi Saitama-ken (JP)**
Inventor: **Araume, Kiyoshi**
**428-2, Miyamae**
**Kounosu-shi Saitama-ken (JP)**
Inventor: **Harayama, Masaaki**
**136-51, Nara-cho**
**Omiya-shi Saitama-ken (JP)**
Inventor: **Nishiyama, Yuichi**
**No. 177-42, Oaza Nishi-Fukushima Kubiki-mura**
**Naka-Kubiki-gun Niigata-ken (JP)**

(74) Representative: **Jaeger, Klaus, Dr.rer.nat. Dipl.-**
**Chem. et al,**
**Jaeger, Grams & Pontani Patentanwälte**
**Bergstrasse 48 1/2**
**D-8035 München-Gauting (DE)**

The file contains technical information
submitted after the application was filed and not
included in this specification

Courier Press, Leamington Spa, England.

**0 035 780**

(56) References cited:

KIRK-OTHMER, ENCYCLOPEDIA OF CHEMICAL TECHNOLOGY Third Edition, vol. 6, 1979 Wiley, New York

CHEMICAL ABSTRACT, vol. 90, no. 8, 19-02-1979, page 387, no. 61181q Columbus, Ohio, USA CHAUMEIL et al. "Gastro-resistant coatings: study of some film-forming agents. Effect of the nature and concentration of plasticizer on cellulose acetophthalate films"

CHEMICAL ABSTRACTS, vol. 89 nr. 24, December 11, 1978 page 409, abstract 204130g Columbus, Ohio, USA DELPORTE et al. "Water vapor permeability of coating materials. Part 1: influence of plasticizers and dispersed solid materials on the water vapor permeability of enterosoluble films: cellulose acetylphthalate"

## Coating compositions for enterosoluble solid dosage forms

Background of the Invention

The present invention relates to an improved coating composition for enterosoluble solid dosage forms or, more particularly, to a composition for providing enterosoluble coating films on the surface of solid dosage forms.

In the prior art, enterosoluble coated solid dosage forms are prepared usually by the method in which the solid dosage forms, e.g. tablets, granules and capsules, are coated with a coating liquid which is a solution of an enterosoluble coating material in an organic solvent. This method of using an organic solution is, however, disadvantageous or defective because the use of a large volume of organic solvents involves, if not to mention the expensiveness of the organic solvents, danger of fire or explosion and health problem of the workers as well as the serious problem of environmental pollution by the dissipation of vapors of the organic solvents into the atmosphere.

Therefore, it has long been desired to develop a method for the preparation of enterosoluble coated medicaments without using organic solvents. An approach in this direction is the use of an aqueous suspension type coating liquid prepared by dispersing finely pulverized water-insoluble film-forming material in water containing a film-forming aid (see Japanese Patent Disclosure 54-84020). When a solid dosage form is coated with such an aqueous suspension and dried by heating, water is evaporated to leave the film-forming material which is converted into coating films on the surface of the dosage form by the aid of the film-forming aid.

A problem in this method is that the film-forming aid must be used in a remarkably large amount relative to the amount of the finely powdered water-insoluble film-forming material or the coating material since otherwise the deposition of the coating material on the solid dosage form is decreased and a large portion of the coating material is carried in the exhaust in the powdery form. Furthermore, the coating films formed with a relatively small amount of the film-forming aid are sometines whitened or exhibit remarkable brittleness.

The film-forming aid used in the above method is selected from the compounds having high solubility in water such as polyethyleneglycol, propyleneglycol, ethyleneglycol monoethyl ether, fatty acid esters of polyoxyethylene sorbitan and the like and the coating films on the solid dosage form are formed, upon evaporation of water in the suspension, by the integration of the film-forming aid in a high proportion with the finely powdered water-insoluble film-forming material. Therefore, the enterosoluble coated solid dosage forms, e.g. tablets, have a problem of premature selective dissolution of the film-forming aid when the coated dosage form is brought into contact with the gastric juice in the stomach. That is, the film-forming aid in the enterosoluble coating film is first leached out into the gastric juice leaving the coting film having a decreased strength so that the solid dosage form per se is sometimes disintegrated in the stomach. Even when no disintegration of the solid dosage form takes place in the stomach, it is unavoidable that a large volume of the gastric juice penetrates into the dosage form so that the effective ingredient in the dosage form is prematurely dissolved out in the stomach or, .vhen the active ingredient is less resistant against the attack of the acidic gastric juice, the ingredient is decomposed to the loss of the effectiveness of the dosage form at all.

In addition, some of the conventionally used film-forming aids such as ethyleneglycol monoethyl ether, propyleneglycol and the like among the above named ones have volatility and the vapors are carried in the exhaust during the coating process to cause environmental pollution.

The inventors have recently proposed an improved method for the preparation of enterosoluble coated solid dosage forms in which the above mentioned problems in the prior art suspension coating method are largely solved. The aqueous suspension here proposed is prepared by dispersing hydroxypropyl methylcellulose phthalate having an average particle diameter of 100 $\mu$m or smaller in water containing triacetin as the film-forming aid and kept at a temperature not to exceed 25°C (see Japanese Patent Disclosure 55-98120).

This method of using a triacetin-containing aqueous suspension is indeed effective in obtaining enterosoluble coated medicaments having good appearance and highly resistant against the attack of the gastric juice to be freed from the problems in the prior art. Unfortunately this method still has a problem, though dependent on the storage conditions, that the triacetin contained in the enterosoluble coating film is gradually hydrolyzed during long-term storage of the dosage form products, especially, in a highly humid, hot atmosphere to liberate acetic acid so that the enterosoluble dosage forms prepared with triacetin and stored for a long time are sometimes unpleasant to the patient by the smell of the acetic acid even though very slight.

The inventors have conducted further investigations by screening numbers of compounds to find a film-forming aid as a substitute for triacetin, which is free from the problem of storage stability and still has the advantages of triacetin in the enterosoluble coating and arrived at the establishment of the present invention.

Finally, C.A. *90* n° 8, 19-02-1979, page 387, 6118y discloses employing tributyl acetylcitrate as a plasticizer for cellulose acetate phthalate. However, aqueous coating suspensions containing cellulose acetate phthalate and tributyl acetylcitrate proved to show only minor spreadability when applied on tablets consisting of usual carrier material such as lactose and corn starch.

Summary of the Invention

The object of the present invention is therefore to provide a novel and improved means for providing enterosoluble coating on the surface of solid dosage forms and, more particularly, to provide a coating liquid used in the suspension coating of solid dosage forms containing a film-forming aid superior to triacetin both in the coating performance and in the stability in a long-term storage.

The coating composition of the present invention for providing enterosoluble coating on the surface of solid dosage forms is an aqueous suspension comprising a powdery enterosoluble coating material having an average particle diameter of 100 μm or smaller as dispersed in an aqueous medium containing triethyl citrate.

Enterosoluble coated solid dosage forms are readily prepared by using the aqueous suspension of the present invention in a coating procedure with no particular difficulty. The enterosoluble coating films thus obtained are highly resistant against the attack of the gastric juice but still rapidly disintegrated in the intestinal juice. The enterosoluble dosage forms have excellent stability in a long-term storage and never produce unpleasant odors even in a highly humid, hot atmosphere as in the case with triacetin.

Detailed Description of the Preferred Embodiments

The enterosoluble coating material to be suspended in the inventive coating composition may be any one of known ones including hydroxypropyl methylcellulose phthalate (HPMCP) and acidic succinyl and acetyl mixed esters of hydroxypropyl methylcellulose (HPMCAS), cellulose acetate phthalate (CAP) and the like having compatibility with triethyl citrate to be plasticized therewith. The coating material should be in finely powdered form having an average particle diameter of 100 μm or smaller and a maximum particle diameter of 200 μm. If necessary, the material is pulverized by a known method and screened to remove coarser particles. Coarser particles of the coating material cannot give a stable suspension when dispersed in an aqueous medium and difficulties are encountered in the formation of the coating films on the surface of the solid dosage forms in addition to the problem of blockage of the spray gun nozzle used in the coating process. Therefore, it is preferable that the average particle diameter of the coating material does not exceed 50 μm.

The coating composition of the present invention is prepared by adding the powdery enterosoluble coating material as such or as a master suspension of high concentration prepared in advance into an aqueous medium containing a desired amount of triethyl citrate with agitation. The sequential order of the addition of triethyl citrate and the powdery enterosoluble coating material may be reversed, if desired, with no particular disadvantages.

The amount of the powdery enterosoluble coating material is preferably in the range from 3 to 20% by weight or, more preferably, from 5 to 15% by weight in the finished coating liquid. Smaller concentrations are undesirable due to the prolonged coating process to obtain desired thickness of the coating film while a coating film having smooth surface is hardly obtained with a coating liquid of higher concentration than above.

Triethyl citrate contained in the coating liquid penetrates into the particles of the powdery enterosoluble coating material as the water is evaporated by heating for drying and serves to give a gelled film of the coating material on the surface of the solid dosage form. In other words, triethyl citrate acts something like a plasticizer. Accordingly, the amount of triethyl citrate is preferably at least 5% by weight or in the range from 5% to 100% by weight based on the enterosoluble coating material in order to fully exhibit the desired plasticizing effect. More preferable amount of triethyl citrate is in the range from 10 to 70% by weight based on the enterosoluble coating material although larger amounts may be used when particular effects are desired. For example, the pliability of the coating film on the solid dosage form is increased by the use of a larger amount of triethyl citrate by virtue of the platicizing effect so that a relatively large amount of triethyl citrate is recommended when solid dosage forms of complicated shapes are to be coated. It is noted that an increased amount of triethyl citrate does not result in the coating films having decreased resistance against the gastric juice or inadequate enterosolubility.

The coating liquid thus prepared is desirably kept at a temperature of 30°C or below throughout the process from the preparation to the coating process including the storage in the feed tank because higher temperatures may cause agglomeration of the particles of the enterosoluble coating material in the coating liquid resulting in decreased homogeneity of the suspension. Care should also be taken to avoid excessive temperature elevation of the coating liquid in the course of transfer from the storage tank to the spray gun of the coating machine because higher temperatures of the coating liquid accelerate the agglomeration of the particles of the enterosolible coating material in the coating liquid leading to eventual blockage of the pipings and the spray gun nozzles.

The coating liquid thus obtained may contain, if necessary, other additive ingredients such as coloring agents, taste improvers, flavorings, a small amount of other conventional plasticizers, water-soluble or water-dispersible polymeric substances, surface active agents which may serve as a defoaming agent and the like. In particular, the use of a surface active agent is desirable when the amount of triethyl citrate exceeds the solubility limit thereof in the aqueous dispersion medium.

The enterosoluble coated solid dosage forms as the objective products of the invention are obtained by performing the coating process with the coating liquid of the invention, in which any one of known coating methods can be applied without particular difficulties. The apparatus for coating may be a pan coater, drum type coating machine, fluidizing coating machine and the like with an accessory of a spraying means such as air spraying and airless spraying.

It is preferable that the coating liquid is continuously agitated during the coating process to avoid settling of the powdery enterosoluble coating material and other optional additive solid ingredients such as pigments suspended in the coating liquid.

The coating amount with the inventive coating composition should be controlled according to the kind and size of the solid dosage forms as well as the desired degree of enterosolubility. It is usually in the range from 3 to 50% by weight of the coating film as solid based on the solid dosage form so as that the coating film on the solid dosage form has a thickness of 5 to 200 $\mu$m.

Prior to coating of the solid dosage form with the inventive coating composition, the solid dosage form may be provided with an undercoating with a coating solution containing another kind of the coating material, e.g a gastrosoluble coating material such as hydroxypropyl methylcellulose. Such an undercoatng is sometimes advantageous, especially, on the solid dosage forms susceptible to wearing or chipping during coating process whereby the overcoating with the inventive coating liquid can be carried out with safety and enterosoluble coating is obtained with a smaller volume of the coating liquid than otherwise.

The coating process of the solid dosage forms with the inventive coating composition may be followed, according to need, by drying, heat treatment, polishing, sugar coating and coating with different kinds of coating materials.

In the following, the present invention is described in further detail by way of examples together with comparative examples, in which parts and % are all given by parts by weight and % by weight.

Example 1

Enterosoluble coating films were provided on the tablets containing lactose and corn starch as the main ingredients and each having a diameter of 9.0 mm and weighing 270 mg. The formulations of the coating liquids A, B and C, the first being for the present invention and the latter two being for comparative purpose, were as follows, in which the enterosoluble coating material was a HPMCP having an average particle diameter of about 10 $\mu$m obtained by pulverizing and screening a commercial product (HP 55, a tradename by Shin-Etsu Chemical Co., Japan).

Coating liquid A: 100 parts of the liquid contained 10 parts of the HPMCP, 3 parts of triethyl citrate and 0.3 part of polysorbate 80 specified in Japanese Pharmacopoeia, the balance being water.

Coating liquid B: 100 parts of the liquid contained 6 parts of the HPMCP, 12 parts of propyleneglycol, 3.6 parts of ethyleneglycol monoethyl ether and 3 parts of talc, the balance being water.

Coating liquid C: 100 parts of the liquid contained 10 parts of the HPMCP, 3 parts of triacetin and 0.3 part of polysorbate 80 specified in Japanese Pharmacopoeia, the balance being water.

The coating procedure of the tablets with the above prepared coating liquid was conducted in a 24-inch Accelacoater manufactured by Manesty Co., England, equipped with an air-spray type spray gun with a nozzle of 1.2 mm diameter and with a tube-type liquid feeder pump. The conditions of the operation of the coating machine were as follows.

Loading amount of the tablets: 10 kg
Temperature of the coating liquid: 21 to 23°C
Feeding rate of coating liquid: 70 g/minute
Flow rate of spraying air: 150 liters/minute
Temperature of air for drying: 65 to 78°C
Temperature of the tablets during coating: 36 to 39°C

The total amount of the coating liquid corresponded to 800 g of the HPMCP, i.e. 8% of the tablets to be coated, for each of the coating liquids A, B and C.

The thus prepared coated tablets had good appearance and almost no recognizable odor irrespective of the formulation of the coating liquid. The disintegrability test of these enterosoluble coated tablets was undertaken in accordance with the procedure specified in the Ninth Revised Japanese Pharmacopoeia with the tablets as prepared with the coating liquids A, B and C and the tablets prepared with the coating liquids A and C followed by polishing with a wax and stored in a sealed glass bottle for 50 days at 40°C. The results of the disintegrability test were as shown in Table 1 below. The coated tablets prepared with the coating liquid C had a slight smell of acetic acid after the above storage test while no unpleasant smell was produced in the tablets prepared with the coating liquid A after the storage test.

TABLE 1

| Tablets | Coating liquid | Disintegration test with | |
| | | The First Solution | The Second Solution |
| As prepared | A | No disintegration | 9 to 10 minutes |
| | B | Rapid and complete disintegration | Rapid and complete disintegration |
| | C | No disintegration | 11 to 13 minutes |
| After 50 days at 40°C | A | No disintegration | 9 to 10 minutes |
| | C | No disintegration | 12 to 14 minutes |

As is understood from the above given results, the enterosoluble coated tablets prepared with the inventive coating liquid A had no problems in the enterosolubility and the storage stability while the tablets prepared with the coating liquid B had poor resistance against the gastric juice to be completely disintegrated in the First Solution. The tablets prepared with the coating liquid C were satisfactory in the storage stability in relation to the enterosolubility but undesirable due to the smell of acetic acid produced in the lapse of time during storage.

Example 2

The enterosoluble coating material used in this example was a pulverized HPMCAS having an average particle diameter of about 10 $\mu$m and a maximum particle diameter of about 30 $\mu$m. The values of the MS for the hydroxypropyl groups and the DS for the methoxyl groups, acetyl groups and succinyl groups were 0.27, 1.85, 0.51 and 0.28, respectively.

Granules were prepared with pancreatin as the principal ingredient and the granules were screened with #12 and #32 Tyler standard sieves to give a portion corresponding to the particle diameter between 500 $\mu$m and 1410 $\mu$m.

A coating liquid was prepared with 10 parts of the HPMCAS, 2.5 parts of triethyl citrate and 0.3 part of polysorbate 80 as specified in the Japanese Pharmacopoeia, the balance to 100 parts of the coating liquid being water.

A Glatt fluidizing coater (model WSG—5, manufactured by Ohkawara Seisakusho, Japan) was loaded with 5 kg portion of the screened granules of pancreatin and coating was performed with the following conditions of the operation of the machine. The amont of the coating liquid used was 1.5 kg as HPMCAS corresponding to 30% of the granules.

Temperature of the coating liquid: 23°C
Feeding rate of the coating liquid: 60 g/minute

Temperature of the fluidizing air: 80°C
Temperature of the exhaust air: 32 to 36°C

The disintegrability of the thus coated enterosoluble granules was tested by the method of disintegrability test for enterosoluble granules specified in the Ninth Revised Japanese Pharmacopoeia to find that the test with the First Solution was cleared and the granules were disintegrated in the Second Solution within 4 to 5 minutes to be quite satisfactory as an enterosoluble medicament.

**Claims**

1. A coating composition for providing an enterosoluble coating film on the surface of a solid dosage form which comprises an enterosoluble coating material insoluble in water and selected from the group consisting of hydroxypropyl methylcellulose phthalate, acidic succinyl and acetyl mixed esters of hydroxypropyl methyl cellulose and cellulose acetate phthalate suspended in an aqueous medium containing a plasticizer in an amount from 5 to 100% by weight based on the enterosoluble coating material, characterised in that said plasticizer is triethyl citrate.

2. The coating composition as claimed in claim 1, characterized in that the enterosoluble coating material insoluble in water is in the form of a fine powder having an average particle diameter not exceeding 100 $\mu$m.

3. The coating composition as claimed in claim 1, characterized in that the amount of the enterosoluble coating material insoluble in water suspended in the aqueous medium is in the range from 3 to 20% by weight based on the amount of the coating composition.

4. The coating composition as claimed in claim 1, characterized in that the amount of triethyl citrate is in the range from 10 to 70% by weight of the amount of the enterosoluble coating material insoluble in water.

5. A method for providing an enterosoluble coating film on a solid dosage form which comprises coating the solid dosage form with an aqueous coating composition of claim 1, characterized by applying the suspension of an enterosoluble coating material insoluble in water as dispersed in an aqueous medium containing triethyl citrate and drying the coating composition to evaporate water.

6. The method as claimed in claim 5 characterised in that the aqueous coating composition is kept at a temperature of 30°C or below before coating the solid dosage form under agitation.

7. An enterosoluble coated solid dosage form comprising a core of the solid dosage form of the effective ingredient and an enterosoluble coating film provided thereon characterized in that said coating film is formed with an enterosoluble coating material plasticized with triethyl citrate as claimed in one of the claims 1 through 4.

**Revendications**

1. Composition d'enrobage pour l'obtention d'un film d'enrobage entérosoluble sur la surface d'une forme de dosage solide, comprenant une matière d'enrobage entérosoluble, insoluble dans l'eau, choisie dans le groupe formé par le phtalate de l'hydroxypropyl-méthylcellulose, les esters mixtes succiniques acides et acétiques de l'hydroxypropyl-méthylcellulose et le phtalate d'acétate de cellulose, en suspension dans un milieu aqueux contenant un plastifiant en une quantité comprise entre 5 et 100% en poids sur la base de la matière d'enrobage entérosoluble, caractérisée en ce que ce plastifiant est du citrate de triéthyle.

2. Composition d'enrobage selon la revendication 1, caractérisée en ce que la matière d'enrobage entérosoluble insoluble dans l'eau se présente sous la forme d'une poudre fine dont le diamètre moyen des particules n'est pas supérieur à 100 $\mu$m.

3. Composition d'enrobage selon la revendication 1, caractérisée en ce que la quantité de matière d'enrobage entérosoluble insoluble dans l'eau, en suspension dans le milieu aqueux, est comprise entre 3 et 20% en poids sur la base de la quantité de composition d'enrobage.

4. Composition d'enrobage selon la revendication 1, caractérisée en ce que la quantité de citrate de triéthyle est comprise entre 10 et 70% en poids par rapport à la quantité de matière d'enrobage entérosoluble insoluble dans l'eau.

5. Procédé pour l'obtention d'un film d'enrobage entérosoluble sur une forme de dosage solide selon lequel on enrobe la forme de dosage solide au moyen d'une composition d'enrobage aqueuse telle que définie dans la revendication 1, caractérisé en ce qu'on applique la suspension d'une matière d'enrobage entérosoluble insoluble dans l'eau sous la forme d'une dispersion dans un milieu aqueux contenant du citrate de triéthyle et, ensuite, on sèche la composition d'enrobage afin d'évaporer l'eau.

6. Procédé selon la revendication 5, caractérisé en ce que la composition d'enrobage aqueous est maintenue à une température égale ou inférieure à 30°C, avant son application sur la forme de dosage solide qui s'effectue en agitant continuellement.

7. Forme de dosage solide entérosoluble comprenant le noyau de la forme de dosage solide constitué par l'ingrédient acitif et un film d'enrobage entérosoluble qui le recouvre, caractérisée en ce que le film d'enrobage est obtenu au moyen d'une matière d'enrobage entérosoluble plastifiée avec du citrate de triéthyle conformément à l'une quelconque des revendications 1 à 4.

**Patentansprüche**

1. Überzugsmasse zur Herstellung eines darmlöslichen Uberzugs auf der Oberfläche einer festen Dosisform, wobei die Beschichtungsmasse ein in Wasser unlösliches darmlösliches Beschichtungsmaterial enthält, und zwar Hydroxypropyl-methylcellulosephthalat, saur Bernsteinsäure- und Essigsäuremischester von Hydroxypropyl-methylcellulose oder Celluloseacetatphthalat suspendiert in einem wässrigen Medium, das einen Weichmacher in einer Menge von 5 bis 100 Gewichtsprozent enthält, bezogen auf das darmlösliche Beschichtungsmaterial, dadurch gekennzeichnet, daß der Weichmacher Triethylcitrat ist.

2. Beschichtungsmasse nach Anspruch 1, dadurch gekennzeichnet, daß das in Wasser unlösliche darmlösliche Beschichtungsmaterial feinpulverig ist und einen mittleren Teilchendurchmesser von nicht größer als 100 $\mu$m aufweist.

3. Beschichtungsmasse nach Anspruch 1, dadurch gekennzeichnet, daß die Konzentration des in Wasser unlöslichen darmlöslichen Beschichtungsmaterials, das im wässrigen Medium suspendiert ist, 3 bis 30 Gewichtsprozent beträgt, bezogen auf das Gewicht der Beschichtungsmasse.

4. Beschichtungsmasse nach Anspruch 1, dadurch gekennzeichnet, daß die Konzentration des Triethylcitrats bezogen auf das Gewicht des in Wasser unlöslichen darmlöslichen Beschichtungsmaterials 10 bis 70 Gewichtsprozent beträgt.

5. Verfahren zur Herstellung einers darmlöslichen Überzugs auf einer festen Dosisform nach Anapruch 1, wobei die feste Dosisform mit einer wässrigen Beschichtungsmasse überzogen wird, dadurch gekennzeichnet, daß die Suspension eines in Wasser unlöslichen darmlöslichen Beschichtungsmaterials dispergiert in einem wässrigen, Triethylcitrat enthaltenden Medium aufgebracht und die Beschichtungsmasse anschließend zur Verdampfung des Wassers getrocknet wird.

6. Verfahren nach Anspruch 5, dadurch gekennzeichnet, daß die wässrige Beschichtungsmasse bei einer Temperatur von 30°C oder darunter gelagert wird, ehe die feste Dosisform unter Rühren mit der Masse überzogen wird.

7. Eine darmlöslich beschichtete feste Dosisform bestehend aus einem Kern in Form einer festen Formulierung des Wirkstoffs, auf der eine darmlösliche Überzugsschicht aufgebracht ist, dadurch gekennzeichnet, daß die Überzugsschicht aus einem darmlöslichen Beschichtungsmaterial hergestellt ist, das nach einem der Ansprüche 1 bis 4 mit Triethylcitrat weichgemacht ist.